# EUROPÄISCHE PATENTSCHRIFT

(11) **EP 1 317 257 B1**
(45) Veröffentlichungstag und Bekanntmachung des Hinweises auf die Patenterteilung: **18.04.2007**
(21) Anmeldenummer: 01960220.0
(22) Anmeldetag: 16.05.2001
(51) Int. Cl.: A61K 9/70

(54) **PFLASTER**
PLASTER
EMPLATRE

(30) Priorität: 17.05.2000 DE 10024396
(43) Veröffentlichungstag der Anmeldung: 11.06.2003
(73) Patentinhaber: Beiersdorf AG, 20245 Hamburg (DE)
(72) Erfinder: CORDES, Günter, 42799 Leichlingen (DE); VOLLMER, Ulrike, 41542 Dormagen (DE)
(86) Internationale Anmeldenummer: PCT/EP2001/005603
(87) Internationale Veröffentlichungsnummer: WO 2001/087274

(56) Entgegenhaltungen:
- WO-A-00/67732
- DE-A- 4 433 666
- DE-A- 19 833 177

## Beschreibung

Hautreizungen, wie sie z. B. durch Stiche von Mücken oder nach Kontakt mit Brennnesseln oder Quallen entstehen, werden stets als unangenehm und lästig empfunden. Bei den in dieser Patentschrift beschriebenen Hautreizungen handelt es sich um sämtliche Reaktionen von leichten Rötungen, Quaddeln, Blasen, nicht jedoch um "krankhafte" Zustände wie hochgradige Sensibilisierungen, die zu allergischem Schock oder Spätausbildung von Granulomen unter dem Bild einer Prurigo führen.

Die hier gemeinten Hautreizungen, die mit dem erfindungsgemäßen Produkt behandelt werden, treten vorzugsweise nach Aufenthalten im Freien, wie sie beim Spielen von Kindern in Wald, Wiese oder Wasserrandbereichen auftreten, aber auch Auftreten bei Reisen im Sommer oder in warme Feriengebiete, oder bei Sportaktivitäten, können aber auch anderweitig erhalten werden. Die oben beschriebenen Hautreizungen sind im medizinischen Sinne folgendermaßen zu interpretieren:

Gemäß der Lehrbuchliteratur (Jung, Dermatologie, 1991) erzeugen geringe Reize an den Nozirezeptoren der Haut Juckreiz, stärkere Reize erzeugen Schmerz. Juckreiz kann eine Reaktion auf chemische, mechanische oder thermische Reize sein. Demzufolge treten Quaddeln/Urtikaria sowohl auf durch Einwirkung chemischer Substanzen (Stiche von Insekten= Histamin bei Mückenstichen, Apamin bei Bienenstichen etc.), Brenn-Nessel etc.), sowie durch allergische Immunreaktion auf chemische Substanzen; durch Druck/Reiben (wie z.B. mit einer Büroklammer oder einem Holzspatel, Reiben feiner Haare) etc., als auch durch Wärme (Sport, Heizkissen), sowie nach Licht (Sonnenbrand) auf. Eine juckende Quaddel/Urtikaria zeigt alle Zeichen einer Entzündung wie Rötung, Schwellung, Wärme, "Schmerz" bzw. Juckreiz und ist auf eine kutane Histaminfreisetzung zurückzuführen. Juckreiz ist nicht vollständig medizinisch erklärt und stellt immer einen subjektiven, teils auch psychologischen Parameter dar.

Es gibt seit Jahren einige erprobte Mittel auf dem Markt. So werden überwiegend kühlende Gele und Stifte eingesetzt, die Antihistaminika neben der Kühlung durch verdunstenden Alkohol oder Wasser enthalten. Ferner wird Isoprenalin in Form von Puder oder Creme verwendet, was auf Dauer genauso wie der Gegenirritator Crotamiton nicht empfohlen wird wegen Hautreizung, abgesehen davon, dass die Wirkung nicht belegt oder rationalisiert werden kann. Der Nachteil dieser topischen Formulierungen ist ihre kurze Wirkweise, sodass ständig die Applikation wiederholt werden muss. Außerdem können sich infolge der ungenügenden Wirkdauer die Stiche infolge Kratzens infizieren. Infizierte bzw. durch Kratzen verletzte Haut heilt wesentlich langsamer und evtl. unter Narbenbildung ab. Dann gibt es die Therapie mit oralen Antihistaminika, die für solche nicht als krankhaft zu bezeichnenden Beeinträchtigungen nicht gerne eingesetzt werden bzw. als Nebenwirkung zu einer geringeren Reaktionsfähigkeit infolge Sedierung führen.

In der Patentschrift DE 19833177 A1 wird beschrieben, dass die Applikation eines Pflasters sehr effektiv gegen die Folgen von Insektenstichen oder Brennnesselkontakt eingesetzt werden kann, wenn eine bestimme Zusammensetzung der Grund- und Hilfsstoffe erfolgt und wenn dafür gesorgt wird, dass die eingesetzten Substanzen längere Zeit Kontakt mit der verletzten/betroffenen Hautstelle haben und gleichzeitig ein Kratzen unterbunden wird. Das darin beschriebene Pflaster enthält eine Kombination von Menthol und einem Lokalanästhetikum vom Estertyp (Benzocain).

Überraschenderweise wurde jetzt in einem biologischen Labortest, in dem doppelblind und placebokontrolliert mit Hilfe von normierten Mückenstichen (Aedes aegypti) verschiedene Formulierungen getestet wurden, festgestellt, dass die Anwesenheit des Lokalanästhetikums vom Estertyp (hier: Benzocain Base) nicht erforderlich ist, bzw. durch einen Zusatz von 1 % Spitzwegerichtinktur ersetzt werden kann, um die gleiche Wirkung zu erreichen. Bei dem biologischen Test mit Aedesaegypti-Mücken (aus pathogenfreier Züchtung der Universität Bonn, Parasitologie) wurden jeweils 2-4 Insekten pro Person mittels eines Glaszylinders auf die Unterarme der 4 freiwilligen Testpersonen aufgesetzt, bis mindestens 1-2 Stiche pro Unterarm erfolgt waren. Sobald die Testperson Juckreiz empfand, wurden die Stiche ausgemessen und mit Pflastern (verblindet und randomisiert) auf beiden Unterarmen beklebt . Die Testpersonen wurden nach ihrer Empfindung 10, 20 und 30 Minauten nach Applikation gefragt. Zwei Stunden nach Applikation wurde das Pflaster entfernt und die Größe der Stichstelle gemessen. Folgende Formulierungen wurden getestet:

| *Formulierung* | *Wirksame Bestandteile (Gehalt in der Klebematrix)* |
|---|---|
| A | Menthol (5%), Benzocain (8%), Polidocanol (8%) |
| B | Menthol (5%), Spitzwegerichextrakt (1%), Polidocanol (8%) |
| C | Menthol (5%) |
| D | Placebo |

Folgendes Ergebnis wurde ermittelt:

| *Formulierung* | *Juckreiz* | *Rötung* |
|---|---|---|
| A | Abnahme nach 10 min, kein Juckreiz mehr nach 20 min | Abnahme des Durchmessers um ca. 0,5 cm |
| B | kein Juckreiz mehr nach 20 min | Abnahme des Durchmessers um ca. 0,5 cm |
| C | Abnahme nach 30 min | teilweise Abnahme des Durchmessers um ca. 0,5 cm |
| D | unverändert nach 30 min | Abnahme des Durchmessers um ca. 0,5 cm |

Da bekanntlich Lokalanästhetika auch in der Lage sind, Kontaktallergien auszulösen bzw. der Zusatz von Benzocain nicht zulässig ist für den späteren Vertrieb als Kosmetikum, wurde die Formulierung ohne Lokalanästethikum speziell für den Einsatz bei Kindern weiterentwickelt.

Bei Kindern ruft die Abdeckung der Reizstelle mit einem Pflaster einen stark psychologisch bedingten Effekt hervor. Dazu muss die Abdeckung ein kindgerechtes, ansprechendes Aussehen besitzen. Dies ist zu erzielen z.B. durch einen Aufdruck beliebter Motive aus dem Comicbereich (Sesamstraße, Puh-Bär, Teletubbies etc.), die sich im Streudruck auf die Trägerfolie aufbringen lassen. Eine andere Möglichkeit besteht in der unifarbenen Einfärbung. Da man dazu mehrere Trägerfolien in verschiedenen Farben beim Folienlieferanten in Auftrag geben, prüfen und vorrätig halten müsste, wurde eine logistisch und finanziell günstigere Variante ermittelt, indem die Pflasterformulierung selber unter Zusatz lebensmittel- und kosmetikgerechter Farbstoffe eingefärbt werden kann. Unter Einsatz einer transparenten Trägerfolie erhält man dann bunte, kindergeeignete Pflasterabdeckungen zur Behandlung von Hautreizungen nach Insektenstichen, Brennnesselkontakt u.ä. Wichtig dabei ist, dass Farben eingesetzt werden, die physiologisch unbedenklich sind, in der Pflasterformulierung in Lösung gehen und keine Färbung auf der Haut beim Tragen hinterlassen. Der Fachmann kann dann nahezu in allen gewünschten Farben (gelb, orange, rot, türkis, grün etc.) Pflaster herstellen. Nicht geeignet ist z.B. der Einsatz von Amaranth, da dieser sich in lösungsmittelbasierten Acrylatklebergrundlagen nicht löst, oder Methylenblau, da dieses beim Tragen die Haut blau verfärbt. Es kann zweckdienlich sein, außer dem Farbstoff zusätzlich Titandioxid zuzugeben, um die Farbe des transparenten Pflasters zu vertiefen. Die beiden folgenden Beispiele beschreiben erfindungsgemäße Formulierungen:

Um 1800 cm² eines erfindungsgemäßen gelben Pflasterlaminats im Labormaßstab herzustellen, werden 1,385 g Menthol,1,440 g Thesit und 4,5 g Spitzwegerichtinktur 4 % , 43,927 g Durotak 87-2852 und 2,3 ml Ethanol 96% gemischt, anschließend 323,5 mg β-Carotin zugegeben und durch Rühren homogenisiert. Die Masse wird mittels eines Rakels in einer Naßschichtdicke von 400 µm auf eine silikonisierte Polyesterfolie ausgestrichen, sodass nach dem Trocken ein Flächengewicht von 100 g/m² erhalten wird. Nach dem Kaschieren mit Cotran # 9720 enthält ein 4,0 cm² großes, leuchtend gelbes Pflaster 1,5 mg Menthol.

Um 1800 cm² eines erfindungsgemäßen roten Pflasterlaminats im Labormaßstab herzustellen, werden 1,385 g Menthol,1,440 g Thesit und 4,5 g Spitzwegerichtinktur 4 % , 43,927 g Durotak 87-2852 und 2,3 ml Ethanol 96% gemischt, anschließend 212,5 mg Eisen-III-oxid zugegeben und durch Rühren homogenisiert. Die Masse wird mittels eines Rakels in einer Naßschichtdicke von 400 µm auf eine silikonisierte Polyesterfolie ausgestrichen, sodass nach dem Trocken ein Flächengewicht von 100 g/m² erhalten wird. Nach dem Kaschieren mit Cotran # 9720 enthält ein 4,0 cm² großes, tomatenrotes Pflaster 1,5 mg Menthol.

Natürlich sind die oben genannten Beispiele auch ohne Farbstoff herstellbar, um sie bei Erwachsenen einzusetzen, die dann durch die transparenten Pflaster den beruhigenden, heilungsfördernden Effekt der Pflaster auf die Hautreizung direkt visuell verfolgen zu können und möglichst dezent und unauffällig auch Hautreizungen im Gesicht kosmetisch akzeptabel lindern zu können.

Darüber hinaus sind auch die dem Fachmann bekannten anderen Pflastergrundlagen wie Heißschmelzkleber, Silikone, Kautschuk und wasserbasierte Systeme einsetzbar, es müssen die Farbstoffe aber jeweils darauf neu abgestimmt werden.

## Patentansprüche

1. Pflasterzubereitung, die aufgrund der Zusammensetzung der Substanzen Menthol, Thesit und Spitzwegerichtinktur eine Reizwirkung, die sich in Kribbeln, Jucken und Brennen äußert, insbesondere nach Brennnessel-Kontakt und Insektenstichen, mindert.

2. Pflasterzubereitung nach Anspruch 1, die bei der Applikation aufgrund einer mit einer selbstklebenden Haftkleberschicht untrennbar verbundenen Abdeckfolie einen Kratzschutz darstellt.

3. Pflasterzubereitung nach Anspruch 1 und/oder 2, **dadurch gekennzeichnet, dass** es sich bei der Zusammensetzung um eine Kombination von Menthol, Thesit und Spitzwegerichtinktur in gelöster Form in einer Haftkleberschicht eines Flächengewichts von 20 bis 200 g/m², vorzugsweise von 80 bis 120 g/m² handelt.

4. Pflasterzubereitung nach mindestens einem der Ansprüche 1 bis 3, **dadurch gekennzeichnet, dass** es sich bei der Zusammensetzung um eine Kombination von Menthol in der Konzentration von 1-10%, vorzugsweise von 3-5%, Thesit in der Konzentration von 2-10 %, vorzugsweise etwa 8 %, und Spitzwegerich in der Konzentration von 0,5-5%, vorzugsweise von 1% handelt.

5. Pflasterzubereitung nach mindestens einem der Ansprüche 1 bis 4, **dadurch gekennzeichnet, dass** die Pflastermatrix durch lebensmittel- bzw. kosmetikzugelassene Farbstoffe eingefärbt ist.

6. Pflasterzubereitung nach mindestens einem der Ansprüche 1 bis 5, **dadurch gekennzeichnet, dass** die Pflastermatrix durch lebensmittel- bzw. kosmetikzugelassene Farbstoffe eingefärbt ist, die in der Matrix löslich sind und die beim Tragen nicht die Haut verfärben.

7. Pflasterzubereitung nach mindestens einem der Ansprüche 1 bis 6, **dadurch gekennzeichnet, dass** die Farbe der Pflastermatrix durch den Zusatz an Titandioxid verstärkt wird.

8. Pflasterzubereitung nach mindestens einem der Ansprüche 1 bis 4 oder bis 7, **dadurch gekennzeichnet, dass** die Abdeckfolie im Streudruck mit Bildern aus der Comicbranche versehen ist.

9. Pflasterzubereitung nach mindestens einem der Ansprüche 1 bis 8, **dadurch gekennzeichnet, dass** die mit der Haftkleberschicht untrennbar verbundene Abdeckfolie eine farblose, flexible Folie ist, die aus Polyethylen, LDPE, EVA-Copolymeren oder Polyurethan, vorzugsweise aus Polyethylen besteht.

10. Pflasterzubereitung nach mindestens einem der Ansprüche 1 bis 6 oder bis 9, **dadurch gekennzeichnet, dass** der Haftkleber ein Heißschmelzkleber aus EVA-Copolymeren und aromatischen Hydrocarbon-Harzen oder ein durch radikalische Polymerisation aus Acrylat-Copolymeren gewonnener Lösungsmittel-Acrylat-Kleber ist.

## Claims

1. Plaster preparation which owing to the composition of the substances menthol, Thesit and tincture of plantain diminishes an irritant effect which is manifested in formications, itches and burns, in particular following contact with stinging nettles and following insect bites.

2. Plaster preparation according to Claim 1 which on application, owing to a cover foil connected inseparably to a self-adhesive layer of pressure-sensitive adhesive, represents a protection against scratching.

3. Plaster preparation according to Claim 1 and/or 2, **characterized in that** the composition is a combination of menthol, Thesit and tincture of plantain in dissolved form in a pressure-sensitive adhesive layer with a basis weight from 20 to 200 g/m², preferably from 80 to 120 g/m².

4. Plaster preparation according to at least one of Claims 1 to 3, **characterized in that** the composition is a combination of menthol in a concentration of 1-10%, preferably of 3-5%, Thesit in a concentration of 2-10%, preferably about 8%, and plantain in a concentration of 0.5-5%, preferably of 1%.

5. Plaster preparation according to at least one of Claims 1 to 4, **characterized in that** the plaster matrix is coloured by means of dyes that are authorized for foods and/or cosmetics.

6. Plaster preparation according to at least one of Claims 1 to 5, **characterized in that** the plaster matrix is coloured by means of dyes that are authorized for foods and/or cosmetics and which are soluble in the matrix and do not, when worn, discolour the skin.

7. Plaster preparation according to at least one of Claims 1 to 6, **characterized in that** the colour of the plaster matrix is intensified through the addition of titanium dioxide.

8. Plaster preparation according to at least one of Claims 1 to 4 or to 7, **characterized in that** the cover foil bears cartoon figures applied using scatter printing.

9. Plaster preparation according to at least one of Claims 1 to 8, **characterized in that** the cover foil joined inseparably to the pressure-sensitive adhesive layer is a colourless flexible foil which is made of polyethylene, LOPE, EVA copolymers or polyurethane, preferably of polyethylene.

10. Plaster preparation according to at least one of Claims 1 to 6 or to 9, **characterized in that** the pressure-sensitive adhesive is a hot-melt adhesive formed from EVA copolymers and aromatic hydrocarbon resins or a solvent acrylate adhesive obtained by means of radical polymerization from acrylate copolymers.

## Revendications

1. Préparation d'emplâtre qui, en raison de la composition en les substances menthol, dodécoxyéthanol (Thesit) et teinture de plantain lancéolé, diminue une irritation qui se manifeste par des picotements, des démangeaisons et des brûlures, en particulier après contact avec des orties et des piqûres d'insectes.

2. Préparation d'emplâtre selon la revendication 1, qui dans l'application représente une protection contre le grattage, en raison d'un film de recouvrement inséparablement lié à une couche autoadhésive d'autoadhésif.

3. Préparation d'emplâtre selon la revendication 1 et/ou la revendication 2, **caractérisée en ce que** la composition consiste en une association de menthol, dodécoxyéthanol (Thesit) et teinture de plantain lancéolé sous forme dissoute dans une couche d'autoadhésif ayant un poids par unité de surface de 20 à 200 g/m², de préférence de 80 à 120 g/m².

4. Préparation d'emplâtre selon au moins l'une des revendications 1 à 3, **caractérisée en ce que** la composition consiste en une association de menthol à une concentration de 1-10 %, de préférence de 3-5 %, de dodécoxyéthanol (Thesit) à une concentration de 2-10 %, de préférence d'environ 8 %, et de plantain lancéolé à une concentration de 0,5-5 %, de préférence de 1 %..

5. Préparation d'emplâtre selon au moins l'une des revendications 1 à 4, **caractérisée en ce que** la matrice de l'emplâtre est colorée par des colorants autorisés pour les produits alimentaires ou les cosmétiques.

6. Préparation d'emplâtre selon au moins l'une des revendications 1 à 5, **caractérisée en ce que** la matrice de l'emplâtre est colorée par des colorants autorisés pour les produits alimentaires ou les cosmétiques, qui sont solubles dans la matrice et qui ne colorent pas la peau lorsque l'emplâtre est porté.

7. Préparation d'emplâtre selon au moins l'une des revendications 1 à 6, **caractérisée en ce que** la couleur de la matrice de l'emplâtre est accentuée par l'addition de dioxyde de titane.

8. Préparation d'emplâtre selon au moins l'une des revendications 1 à 4 ou à 7, **caractérisée en ce que** le film de recouvrement est muni, dans l'impression continue, d'images provenant de bandes dessinées.

9. Préparation d'emplâtre selon au moins l'une des revendications 1 à 8, **caractérisée en ce que** le film de recouvrement inséparablement lié à la couche d'autoadhésif est un film souple incolore qui est constitué de polyéthylène, LPDE, copolymères EVA ou polyuréthanne, de préférence de polyéthylène.

10. Préparation d'emplâtre selon au moins l'une des revendications 1 à 6 ou à 9, **caractérisée en ce que** l'autoadhésif est un adhésif fusible à base de copolymères EVA et de résines hydrocarbonées aromatiques ou un adhésif acrylate-solvant obtenu par polymérisation radicalaire à partir de copolymères d'acrylate.
